# EUROPEAN PATENT APPLICATION

(11) **EP 4 008 707 A1**
(43) Date of publication of application: **08.06.2022**
(21) Application number: 20211706.5
(22) Date of filing: 03.12.2020
(51) Int. Cl.: C07C 29/12, C07C 31/20, C07C 303/24, C07C 305/10, C07D 327/10

(54) **PROCESS FOR THE CONVERSION OF ETHANE TO ETHYLENE GLYCOL**

(71) Applicant: Studiengesellschaft Kohle mbH, 45470 Mülheim (DE)
(72) Inventor: SCHUETH, Ferdi, 45470 Mülheim an der Ruhr (DE); BILKE, Marius, 47829 Krefeld (DE); ZIMMERMANN, Tobias, 69123 Heidelberg (DE)

(57) **Abstract**

The present invention refers to an attractive route for the conversion of ethane to ethylene glycol. In particular, the present invention includes a process for the production of ethylene glycol from shale gas.

## Description

The present invention refers to an attractive route for the conversion of ethane to ethylene glycol. In particular, the present invention includes a process for the production of ethylene glycol from shale gas and other ethane-rich gases.

Ethane is one of the least reactive, but at the same time one of the most abundant hydrocarbons. It is present in many natural gas resources, but particularly its significant share in shale gas resources makes ethane an increasingly attractive feedstock for the chemical industry. On a technical scale ethane is almost exclusively used for the production of ethylene in the highly endothermic steam cracking process, being expensive in terms of energy consumption and investment costs. Conversion to oxy-functionalized products in any case requires additional processing steps.

Although numerous strategies have been pursued to directly oxidize ethane under mild conditions, hardly any of these have passed the pilot stage yet. Proposed reaction schemes typically suffer from severely limited product selectivity or the dependence on activated oxidants, such as high-valency main group element compounds or hydrogen peroxide (Catalysts 2016, 6, 71). Particularly promising are routes from ethane to vinyl chloride combining chlorination and oxychlorination reactions, and a direct partial oxidation process towards acetic acid as disclosed in US6797845. The process as disclosed in Chemical Reviews 2017, 117, 4182-4247 has even been commercialized by SABIC; however, its economic viability compared to the well-established Cativa process is certainly questionable.

Another strategy that has shown promise for low-temperature short-chain alkane functionalization relies on molecular catalysts with C-H bond activation as the working principle (Angewandte Chemie International Edition 2011, 50, 10096-115). Here, the "Periana/Catalytica" system, that has attracted great interest, utilizes sulfuric acid or oleum as the transfer oxidant and is known as an efficient system for the selective conversion of methane toward methyl bisulfate (MBS) (Science 1998, 280, 560-4). Already at an early stage, it was demonstrated that the sulfuric acid system can in principle also be used for the functionalization of higher alkanes under relatively mild conditions. At temperatures as low as 90 °C the conversion of ethane to ethionic acid (ETA) is observed - even in the absence of a catalyst.

Periana et al. (Journal of the American Chemical Society 2014, 136, 10085-94) built on these findings and more recently described the efficient and selective C-H functionalization of ethane to ethionic acid (HO₃S-CH₂-CH₂-OSO₃H, ETA) using Pt(bpym)Cl₂ at 160 °C in concentrated sulfuric acid.

In a previous report, molecular iodine was described as an appropriate catalyst for the oxidation of short-chain alkanes to the corresponding esters in oleum (Chemical Communications 2002, 2376-7). The results obtained by Periana et al. however suggested oleum to be inappropriate for the functionalization of alkanes higher than methane, since undesired side reactions were observed. Similar findings inspired Bjerrum et al. to even file the patent application WO9924383, claiming the functionalization of any gaseous aliphatic hydrocarbon in oleum. However, all the examples described therein are limited to reactions of methane. According to Periana et al., iodine-mediated reactions of "higher alkanes" - which also comprise the next homologue ethane - in oleum proceeded with a significantly lower product selectivity than in case of methane. Although no further details were disclosed, it was concluded, that proton catalyzed reactions in fact make higher alkanes unsuitable substrates in oleum. In essence, the catalytic use of hypervalent iodine for the *selective* oxidation of short-chain alkanes in sulfuric acid/oleum was thus far limited to methane as the substrate.

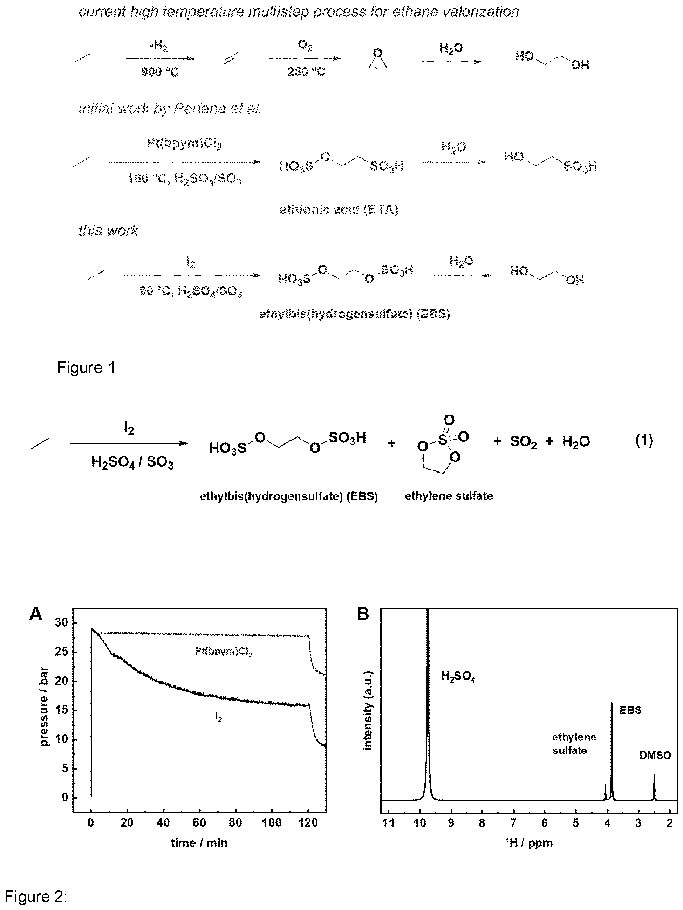

After thoroughly investigating this catalytic system in a broad range of reaction conditions, the inventors here have found that the use of catalytic amounts of iodine is suitable for the selective functionalization of ethane in oleum. The present iodine system surpasses the previously reported Pt-based system not only in terms of productivity, but furthermore the selectivity is altered in a way that the easily hydrolysable bis-bisulfate ester of ethylene glycol is obtained as the only product. Thus in first test runs, the present inventors have surprisingly found the outcome of an initial test reaction of ethane with a 5 mM solution of iodine in 20% oleum, that was conducted at a substantially reduced reaction temperature of 90 °C to compensate for the increased reactivity of the substrate: Within the course of 1 h reaction time the pressure decreased from initially 30 to almost 15 bar - being a consequence of ethane conversion to liquid products (see Figure 2A). ¹H NMR analysis of the liquid phase revealed the bis-bisulfate ester of ethylene glycol (EG(OSO₃H)₂, EBS) along with ethylene sulfate ((CH₂O)₂SO₂) as the only reaction products (X∼60%, S > 98%, see Figure 2B and eq. (1)). As in almost all experiments of this study, the carbon balance closed to well above 90%.

Thus, the present invention relates to a process for producing an ethylene glycol (1,2-ethanediol) from gaseous ethane, especially from shale gas, via oxidative reaction of ethane in a concentrated sulfuric acid medium in the presence of a catalytic amount of a iodine compound as catalyst. In the process, the reaction takes place in a pressurizable reaction vessel. Stirring or other effective mixing of the reaction medium during the reaction is generally to be preferred. The pressure in the pressurizable reaction vessel is usually in the range from more than atmospheric pressure of 1 bar, preferably more than 10 bars, up to a high pressure with an optional upper limit of the vapor pressure of ethane at the reaction temperature in the area around the critical pressure of approx. 48,7 bar. Alternatively, the reaction can also be carried out at pressures at which ethane is in the supercritical state. The catalyst is usually selected from elemental iodine, a iodine compound or a mixture thereof,

In more detail, the present invention provides a process for producing an ethylene glycol derivative from ethane via an oxidative reaction of ethane in a concentrated sulfuric acid medium in the presence of a catalyst in a pressurizable reaction vessel, wherein:
- the concentrated sulfuric acid medium is oleum having a sulfuric acid-sulfur trioxide-concentration in a range of 19 to 22 mol/L,
- the catalyst, which is selected from elemental iodine, a iodine compound or a mixture thereof, is present in the reaction mixture in a catalytic amount of 5 to 50 mmol/L,
- the reaction pressure is in a range of more than 10 bar, and
- the reaction temperature is in a temperature range of 60°C to 150°C,
whereby a reaction product is obtained.

In further embodiments of the inventive process for producing an ethylene glycol from ethane:
- the concentrated sulfuric acid medium is oleum having a sulfuric acid-sulfur trioxide-concentration in the range of 19 to 21 mol/L, and/or
- the catalyst is used in a catalytic amount of 10 to 30 mmol/L, and or
- the reaction pressure is in the range of more than 20 bar, and/or
- the reaction temperature is in a temperature range of 70°C to 130°C.

In order to form the desired glycol from the product or products of the reaction of ethane in the sulfuric acid medium, the product, e.g. an ethylene sulfate or ethyl bis(hydrogensulfate), is suitably subjected to an hydrolysis treatment, e.g. using water or an aqueous medium. After hydrolysis, the obtained glycol is separated from the reaction mixture, preferably by distillation. Alternatively, the obtained product such as ethylene sulfate or ethyl bis(hydrogensulfate) can be separated by distillation, membrane separation, or another suitable method prior to hydrolysis. Hydrolysis of ethylene sulfate or ethyl bis(hydrogensulfate) can then proceed in a separate vessel. This has the advantage of avoiding dilution of the oleum.

The sulfuric acid medium employed in the process preferably contains dissolved SO₃ in an amount of from 10% by weight up to the solubility limit of SO₃ in H₂SO₄ under the particular reaction conditions (temperature, pressure, etc.) employed.

The SO₃ content of the sulfuric acid will, however, generally be in the range of 10 to 65% by weight (% w/w), and normally in the range of 30 to 65% w/w. This corresponds to an oleum having a sulfuric acid-sulfur trioxide-concentration in the range of 19 to 23,5 mol/L, preferably of 20 to 22 mol/L.

The reaction temperature in the sulfuric acid medium will normally be in the range of 60°C to 150°C, preferred 80°C to 125°C and further preferred from 90°C to 120°C.

With respect to pressure conditions, the reaction in the sulfuric acid medium will take place in a pressurizable reaction vessel into which ethane is introduced to an initial pressure in the range of 1-500 bar. It is, however, generally preferable to employ an initial pressure in the range of 10 to 50 bar, such as in the range of 20 to 40 bar.

The added catalyst employed is elemental iodine in an amount of 5 to 50 mmol/L, preferably 10 to 30 mmol/L. Also other iodine compounds, especially high valence compounds such as I₂O₅ or KIO₄, but even iodides can be used as catalysts in similar concentration ranges.

In an embodiment of the invention, ethane is provided to the reaction vessel in form of shale gas.

The present invention is further illustrated by the attached Figures and Examples. In the attachment, the Figures show:
Figure 1: Comparison of the industrial state-of-the-art pathway for ethane valorization with previous work by Periana et al. and the present invention;
Figure 2:
   (A) Pressure-time curve indicating proceeding ethane conversion to liquid products (conditions: 15 mL of a 5 mM catalyst solution in 20% oleum, 90 °C).
   (B) Representative ¹H NMR spectrum of a post-reaction mixture showing signals for sulfuric acid as well as EBS and ethylene sulfate.
Figure 3:
   A) Conversion (■) and combined selectivity (•) towards EBS and ethylene sulfate as a function of sulfur trioxide concentration at 90 °C. 15 mL of a 5 mM [I] solution in sulfuric acid/oleum, initial pressure 30 bar, reaction stopped after 2 h.
   (B) TOF (■) in mol_{product} mol⁻¹_{catalyst} h⁻¹ and combined selectivity (•) towards EBS and ethylene sulfate in % for as a function of sulfur trioxide concentration at 90 °C. 15 mL of a 5 mM [I] solution in sulfuric acid/oleum, initial pressure 30 bar, conversion of ethane < 30%.
   (C) Conversion (■) and combined selectivity (•) towards EBS and ethylene sulfate in % as a function of temperature. 15 mL of a 5 mM [I] solution in 20% oleum, initial pressure 30 bar, reaction stopped after 2h.
   (D) Rate in mol_{product} h⁻¹ (■) and combined selectivity (•) towards EBS and ethylene sulfate in % as a function of catalyst concentration at 90 °C. 15 mL of a catalyst solution in 20% oleum, conversion of ethane < 30%.

As shown in Figure 1, the process of the present invention can be carried out under less drastic conditions compared to the industrial state-of-the-art pathway for ethane valorization with previous works of the Periana group.

As illustrated in Figure 2, the subsequent hydrolysis of the reaction solution (water added in 4-fold volumetric excess, 90 °C, 3 h) gives a mixture of ethylene glycol and the corresponding mono- and bis-bisulfate esters in a ratio of 1: 0.33: 0.01. Control reactions under the same conditions as mentioned above showed no ethyl products in the absence of iodine. Contrary to generalized assumptions from an earlier report, iodine can indeed act as a highly active catalyst for the selective ethane functionalization in oleum under well-chosen conditions. For comparison the "Periana/Catalytica" catalyst was studied under the same reaction conditions. Here, almost no change in pressure was observed within the same period of time (Figure 2A). It is notable, that under optimized conditions, i.e. with a lower oleum concentration of 1.5 wt.%, albeit an increased reaction temperature of 160 °C, Pt(bpym)Cl₂ yielded only about one fourth of a valuable product (ETA) compared to iodine (EBS) in 20% oleum at 90 °C.

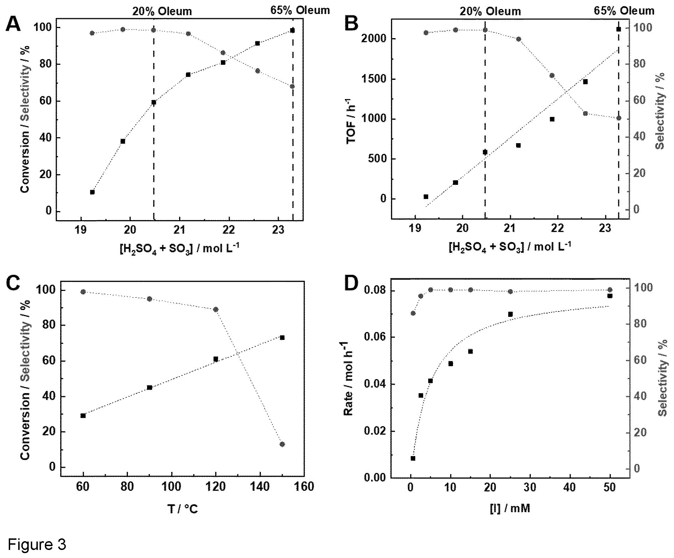

As illustrated in Figure 3, the inventors investigated in a systematic manner, if a comparable behavior is observed for the iodine-catalyzed functionalization of ethane, with an emphasis on higher oleum concentrations. Figure 3A displays ethane conversion and selectivity toward the desired products as a function of SO₃ concentration. While in concentrated sulfuric acid the inventors could not find any ethyl products, already at relatively low oleum concentrations ethane is cleanly converted to the bis-bisulfate ester of ethylene glycol. Ethane conversion increases steadily over the whole concentration range, and in some cases almost full conversion could be achieved. Performing the reaction at a reduced reaction temperature allows the use of this more strongly oxidizing reaction medium without sacrificing product selectivity. Selectivity, however, suddenly drops when [H₂SO₄ + SO₃] reaches values higher than 21 M. Above that level, ETA is observed as a side product. Furthermore, increasing amounts of CO₂ were detected in the gas-phase, which indicates that consecutive reactions of the primary oxidation product take place.

For increasing reaction temperatures increasing ethane conversion is observed (Figure 3C), as expected. However, at the same time there is a strong decline in selectivity, which is particularly pronounced for temperatures above 120 °C. E.g. at 150 °C roughly 8 times more CO₂ is detected compared to the experiment at 90 °C. In this particular case only 50% of the carbon atoms could be recovered as carbon dioxide or EBS. Although other potential side products were not identified, a very similar behavior is known from surfactant synthesis, where oleum is used for the sulfonation of fatty alcohols. Efficient heat removal is essential to avoid so-called "product darkening", being a consequence of undesired decomposition reactions. Thermal treatment of solutions of independently synthesized EBS in 20% oleum revealed product stability at 90 °C, whereas at a temperature of 150 °C almost complete decomposition was observed within 2 h. One possible decomposition product could be vinylsulfonic acid, which might form oligomeric species in the presence of radicals.

In analogy to a similar study of the iodine-catalyzed methane functionalization, also for ethane a steadily increasing reaction rate with increasing catalyst concentration is observed (see Figure 3D). Above a certain concentration level the curve is flattening. This is most probably due to the fact that the solution is saturated with iodine from this point on. In other words, the solubility of iodine can be estimated to be 40 mM which is in the same range as values reported for comparable systems. Moreover, depending on the exact catalyst concentration, different cationic iodonium species might be present for a given SO₃ concentration. The differences in color that were observed for post-reaction mixtures might hint at speciation coming into play. In-depth studies are required to determine the nature of the active species and their intrinsic catalytic properties.

To examine similarities between the reactions with methane and ethane, the inventors further performed a competition reaction with a 9:1 mixture of both hydrocarbons, resembling a composition that is typical for shale gas deposits. At 90 °C in 20% oleum EBS was observed as the single product without any C₁ oxygenates being present at all after 2 h. This is unexpected when considering previously reported rates of electrophilic C-H activation reactions of various hydrocarbons, and it points towards substantial differences in the underlying reaction pathways. There have been concerns about the need for separation of higher alkanes, particularly ethane, from natural gas streams before the major component methane is actually functionalized. This is due to the fact that for methane functionalization typically more severe conditions are applied, at which ethane would mostly undergo total oxidation and thus be lost. Due to the reduced reaction temperature, the iodine-catalyzed ethane functionalization the inventors report here is thus a viable strategy to separate the higher alkanes from the methane stream by simultaneous valorization.

### Experimental Part

### Analytical Methods

Elemental analyzes were performed by the Microanalytical Laboratory Kolbe, Mülheim an der Ruhr.
¹H-NMR spectra of reaction solutions were measured at room temperature on an AV-300 spectrometer from Bruker. Chemical shifts are reported in parts per million (ppm) and can vary widely depending on the acidity of the medium. The residual proton signal of DMSO-d⁶ that was added to the sample in a coaxial capillary was taken as a reference. For quantitative evaluation of the catalytic experiments, a pre-weighed amount of methanesulfonic acid (MSA) was added as standard to a defined volume of the reaction solution.

Gas-phase infrared spectra were recorded on a Nicolet Avatar 370 FT-IR spectrometer from Thermo Scientific. For each analysis, 32 scans were measured at a resolution of 2 cm⁻¹. Before quantification of the spectra by integration, the background spectrum was subtracted and an automatic baseline correction was applied. Differences in the extinction of the individual components in the gas phase were accounted for by corresponding correction factors obtained by previous calibration with pure gases (CO₂ 1, C₂H₆ 3.72, CH₄ 11.38).

### Reactor Material and Equipment

Catalytic tests were carried out in pressure- and temperature-resistant autoclaves, which were designed and manufactured in house. Hastelloy G-35 (Ni, 33% Cr, 8% Mo, ≤ 2% Fe, ≤ 0.6% Si, ≤ 0.5% Mn, ≤ 0.4% Al, ≤ 0.05% C) or 1.4571 stainless steel was used as construction material. To further avoid extensive corrosion, glass or Teflon inlets, minimizing the contact of the reaction mixture with the reactor walls, were applied. Whenever necessary, reactors were remachined. Sealing materials consisted of either teflon or gold. All reactors were equipped with pressure meters (JUMO dTRANS p30) and a thermocouple (type K), which was additionally covered by a teflon jacket. Valves and connections made from stainless steel were replaced if necessary.

### General Catalytic Experiment

Catalytic ethane oxidation was carried out in a two-autoclave setup. Both autoclaves were connected via two valves and a short capillary. One autoclave served as a heated C₂H₆ reservoir. The other autoclave, denoted the "reactor", was used for the actual reaction. The C₂H₆ reservoir was filled with ethane to a pressure of 30 bar at room temperature. The reactor was filled with sulfuric acid or oleum and catalyst and purged with argon before closure. Both autoclaves were heated to the desired reaction temperature, and the reaction was started by pressurizing the reactor with preheated ethane. The reaction was run until a certain pressure drop - a consequence of the formation of reaction products, which are liquid under the given conditions - was reached or, in the case of slow rates, for a certain time, typically for 2 h. The conversion of ethane was thus limited to approximately 30%, which ensures comparison of the results of different runs under fairly similar conditions and maintains the SO₃ concentration at a sufficiently high level. The reaction was stopped by quenching the autoclave in a water bath with stirring. The relative composition of the gas phase was analyzed by IR spectroscopy. Liquid-phase analysis was done by means of ¹H NMR spectroscopy of a reaction solution aliquot. TOFs were calculated by dividing the molar amount of products formed by the molar amount of catalyst present in the reactor and by the reaction time. TOFs should ideally be determined from the initial slope of product formation, but due to associated experimental difficulties, determination of an average TOF was chosen instead. This does not necessarily reflect intrinsic activity, and values are apparent, especially for high catalyst loadings, if solubility is low.

### Summary

Direct valorization of ethane, a substantial component of shale gas deposits, at mild conditions remains a significant challenge, both from an industrial and an academic point of view. Herein, the inventors have reported iodine as an efficient and selective catalyst for the functionalization of ethane in oleum at low temperatures and pressures. A thorough study of relevant reaction parameters revealed iodine to be remarkably more active than the previously reported "Periana/Catalytica" catalyst under optimized conditions. As a result of a fundamentally different catalytic cycle, iodine yields the bis-bisulfate ester of ethylene glycol (HO₃SO-CH₂-CH₂-OSO₃H, EBS), whereas for state-of-the-art platinum-based catalysts ethionic acid (HO₃S-CH₂-CH₂-OSO₃H, ETA) is obtained as the main product.

Thus, the inventors here disclose molecular iodine or a iodine containing compound as a highly efficient catalyst for the selective functionalization of ethane in oleum which is in contrast to what has earlier been reported in literature. The bis-bisulfate ester of ethylene glycol (EBS) can be cleanly generated at temperatures as low as 90 °C with reaction rates substantially exceeding those obtained with previously reported Pt-based catalysts. Since the rate of ethane oxidation to EBS substantially exceeds the rate of methane oxidation under appropriate reaction conditions, the finding provides a pathway for the selective conversion of ethane in an excess of methane, as present in shale gas. The inventors could show that hydrolysis of the post-reaction mixture mainly yields ethylene glycol. Thus, the findings of the inventors serve as a basis for a facile two-step process for the production of ethylene glycol from shale gas, bypassing the energy-intensive step of ethylene production that is commonly applied on a technical scale.

## Claims

1. Process for producing an ethylene glycol derivative from ethane via an oxidative reaction of ethane in a concentrated sulfuric acid medium in the presence of a catalyst in a pressurizable reaction vessel, wherein:
- the concentrated sulfuric acid medium is oleum having a sulfuric acid-sulfur trioxide-concentration in a range of 19 to 22 mol/L,
- the catalyst, which is selected from elemental iodine, an iodine compound or a mixture thereof, is present in the reaction mixture in a catalytic amount of 5 to 50 mmol/L,
- the reaction pressure is in a range of more than 10 bar, and
- the reaction temperature is in a temperature range of 60°C to 150°C,
whereby a reaction product is obtained.

2. Process for producing an ethylene glycol derivative from ethane according to claim 1, wherein:
- the concentrated sulfuric acid medium is oleum having a sulfuric acid-sulfur trioxide-concentration in the range of 19 to 21 mol/L, and/or
- the catalyst is used in a catalytic amount of 10 to 30 mmol/L, and or
- the reaction pressure is in the range of more than 20 bar, and/or
- the reaction temperature is in a temperature range of 70°C to 130°C.

3. A process according to claim 1 or 2, wherein said sulfuric acid medium is stirred during said oxidative reaction.

4. Process for producing an ethylene glycol from ethane according to any one of the preceding claims, wherein the obtained reaction product is subjected to a hydrolysis treatment and the hydrolysed product is separated from the reaction mixture, preferably by distillation or membrane separation.

5. Process for producing an ethylene glycol from ethane according to any one of the claims 1 to 3 wherein the obtained reaction product is separated from the reaction mixture, preferably by distillation or membrane separation, and the separated reaction product is subjected to a hydrolysis treatment.

6. A process according to any one of claims 1 to 5, wherein the ethane is provided to the reaction vessel in form of shale gas.
